Europäisches Patentamt

European Patent Office

Office européen des brevets

(11) Numéro de publication: **0 279 779**
**A1**

# (12) DEMANDE DE BREVET EUROPEEN

(21) Numéro de dépôt: 88810078.1

(22) Date de dépôt: **10.02.88**

(51) Int. Cl.⁴: **A 61 N 1/42**
**A 61 N 5/02**

(30) Priorité: 13.02.87 CH 552/87

(43) Date de publication de la demande:
24.08.88 Bulletin 88/34

(84) Etats contractants désignés:
AT BE CH DE ES FR GB GR IT LI LU NL SE

(71) Demandeur: DONEXY S.A.
Rte de Buchillon 8
CH-1162 Saint-Prex (CH)

(72) Inventeur: De Mesnard, Louis-Emmanuel
Virazeil
F-47200 Marmande (FR)

(74) Mandataire: Hranitzky, Wilhelm Max et al
c/o WILLIAM BLANC & CIE Conseils en Propriété
Industrielle SA 6, rue de la Grotte
CH-1003 Lausanne (CH)

(54) Dispositif pour la production de champs magnétiques et électromagnétiques biologiquement actifs.

(57) Le dispositif comprend un générateur (5) d'ondes électro-magnétiques de haute fréquence, un magnétron (9) du type à impulsions à fréquence de relaxation variable, de longueurs d'ondes centimétriques et un générateur (16) de champs magnétiques utilisant une technologie des bobines de Helmoltz (15,15'). Le générateur (5) d'ondes électromagnétiques et le générateur (16) de champs magnétiques sont agencés pour produire des champs croisés ou parallèles, le mélange des champs étant réalisé directement au niveau du corps (2) à traiter.

FIG.1

EP 0 279 779 A1

**Description**

# DISPOSITIF POUR LA PRODUCTION DE CHAMPS MAGNETIQUES ET ELECTROMAGNETIQUES BIOLOGIQUEMENT ACTIFS

La présente invention concerne un dispositif pour la production de champs magnétiques et électromagnétiques biologiquement actifs, dont les effets sont appliquables au corps humain et aux corps de divers animaux à des fins thérapeutiques, ou préventives par stimulation des défenses naturelles de l'organisme.

On a déjà conçu et utilisé divers appareils et procédés utilisant la production d'un champ magnétique ou électromagnétique, dans le but de traiter des affections variées. Un dispositif permettant d'obtenir une combinaison de rayonnement de particules chargées électriquement et d'un rayonnement électromagnétique centimétrique pour pénétrer intimement et irradier des tissus vivants est descrit dans le brevet français no 1 342 772. Ce dispositif permet d'émettre dans une cavité un rayonnement de particules chargées électriquement auquel on superpose un rayonnement électromagnétique centimétrique. Le rayonnement de particules chargées est accéléré dans un accélérateur de particules, le rayonnement résultant sortant de la cavité étant dirigé sur la cible par l'intermédiaire d'un tube à plasma qui est le siège de champs électriques et de champs magnétiques.

Les résultats obtenus avec le dispositif du brevet français no 1 342 772 sont généralement admis comme étant satisfaisants. Toutefois, de par sa conception très complexe et très volumineuse, le dispositif décrit dans ce brevet se révèle d'une réalisation extrêmement coûteuse. D'autre part, le rayonnement étant canalisé dans un tube à plasma, ce dispositif ne permet de traiter le corps humain que par petites zones. De plus, l'énergie utilisée est très importante, pour un rendement faible.

Le but de la présente invention est de permettre la réalisation d'un dispositif de structure simple, peu volumineux et très maniable, susceptible d'être monté le cas échéant sur un support orientable, ce dispositif ne comportant que le minimum d'éléments capables de produire des champs magnétiques et électromagnétiques reconnus comme actifs dans l'état actuel de l'expérimentation biologique; ce qui permet un taux de défaillance de l'appareillage particulièrement faible et une mise en main plus rapide. Un autre but de l'invention est d'assurer l'alimentation des générateurs dans des conditions de rendement énergétiques optimum, par des dispositifs de tension variable, afin d'utiliser seulement la puissance nécessaire pour la thérapie. Dans le cadre du tiers-monde notamment, un simple groupe électrogène est suffisant pour le fonctionnement correct du dispositif.

Outre ses vertus thérapeutiques, le dispositif de l'invention constitue un outil de recherche privilégié dans l'étude des moyens physiques destinés à la stimulation des défenses naturelles de l'organisme et particulièrement dans le renforcement du pouvoir immunitaire des personnes contre les agressions par des agents pathogènes, sans pour autant que le domaine d'usage de l'invention soit limité à cette application particulière, d'autres essais intéressants ayant pu être effectués dans les traitements psychothérapiques, dans d'autres affections et la traumatologie.

A cet effet, l'invention concerne un dispositif pour la production de champs magnétiques et éleotromagnétiques biologiquement actifs caractérisé en ce qu'il comprend un premier générateur d'ondes électromagnétiques de haute fréquence sur un large spectre, un second générateur d'ondes électromagnétiques utilisant les longueurs d'ondes centimétriques et un générateur de champs magnétiques, le premier générateur d'ondes électromagnétiques et le générateur de champs magnétiques étant disposés et agencés pour produire des champs croisés ou parallèles en fonction de la thérapie à appliquer, le mélange des champs étant réalisé directement au niveau du corps à traiter.

Selon un mode d'exécution préféré de l'invention, le dispositif comporte un support destiné à recevoir le corps de la personne ou de l'animal à traiter, en dessous duquel est disposée une électrode neutre, reliée à une borne du générateur de haute fréquence, au moins une électrode active disposée sur un support mobile étant reliée à l'autre borne dudit générateur.

Le deuxième générateur peut être un magnétron du type à impulsions à fréquence de relaxation variable, de longueurs d'ondes centimétriques, le signal dudit générateur étant envoyé sur un système dispersant qui le renvoie sur le corps à traiter, le générateur de champs magnétiques étant disposé de façon à produire un champ magnétique sensiblement constant dans tout le volume du corps à traiter.

Selon une variante d'éxecution, le deuxième générateur et le générateur de champs magnétiques sont solidaires d'un chariot pouvant se déplacer dans une direction parallèle à l'axe longitudinal du support, de façon à produire des champs perpendiculaires au dit axe longitudinal du support.

Le générateur de champs magnétiques peut comporter des aimants placés de part et d'autre du support, de façon à constituer un champ magnétique, au moins un des aimants étant mobile sous l'effet d'un moteur dont le nombre de tours est réglable en fonction de la thérapie à appliquer, ledit générateur étant agencé de façon à produire un champ magnétique faible destiné à être enregistré par les récepteurs magnétiques du corps. En effet ces récepteurs permettent de transmettre au cerveau des informations qui le font réagir en conséquence.

Selon une variante du dispositif, le générateur de champs magnétiques comporte des bobines placées de part et d'autre du support et reliées à un générateur basse fréquence permettant la production d'un champ magnétique variable en fréquence et en intensité.

Le champ magnétique produit peut être continu et sensiblement uniforme de façon à permettre l'orientation des molécules polaires dans un sens privilégié et déterminé en fonction de la thérapie à appliquer. Ceci n'est qu'une analogie par rapport à la résonance magnétique nucléaire où seuls les protons sont mis en résonance à des fins d'imagerie médicale. Ici la résonance moléculaire permet une amélioration des réactions biochimiques dans l'organisme et son effet persiste quelques heures après la séance.

Les éléments qui composent le dispositif peuvent être disposés dans une cage de Faraday.

Le générateur de haute fréquence peut être un oscillateur qui génère une onde haute fréquence amortie croissante et décroissante, deux impulsions successives étant séparées par une zone de non activité de longueur réglable, la zone de non activité pouvant être de longueur nulle.

Le dispositif de l'invention constituant également un outil de recherche thérapeutique, le dispositif peut être complété par une élaboration plus complète de la structure du générateur de champs magnétiques. Des unités de visualisation et d'enregistrement des impédances présentées par le patient permettent une surveillance et une analyse fine des effets de la thérapie en cours de la séance et après cette dernière, afin de constituer un dossier. Dans ce but, des fiches de synthèses peuvent être prévues pour faciliter au mieux les manipulations et le collectage des résultats obtenus par le dispositif, ce qui permet au praticien de déterminer le nombre de séances et leur durée au vu des premières synthèses obtenues par lesdites fiches.

Dans le cadre de la recherche fondamentale, on peut doter les générateurs de paramètres variables quant à la fréquence, l'amplitude et la forme d'ondes, et une imagerie peut être associée afin de voir la répartition dans le corps des différentes impédances présentées aux ondes électromagnétiques. Le tout peut être géré par un ordinateur.

L'invention sera bien comprise à l'aide de la description qui suit, donnée à titre d'exemple, et se référant aux dessins, sur lesquels :

la fig. 1 représente une vue schématique de côté d'un exemple de dispositif selon l'invention, le patient à traiter étant couché sur un lit;

la fig. 2 représente les lignes de champs produites par le premier générateur d'ondes électromagnétiques représenté à la figure 1;

la fig. 3 montre un exemple d'onde haute fréquence amortie croissante et décroissante que l'on produit avec le générateur haute fréquence du dispositif de l'invention;

la fig. 4 montre un exemple d'électrode active utilisée avec le générateur haute fréquence, en vue partielle de dessus (fig. 4a) et en vue partielle de profil (fig. 4b).

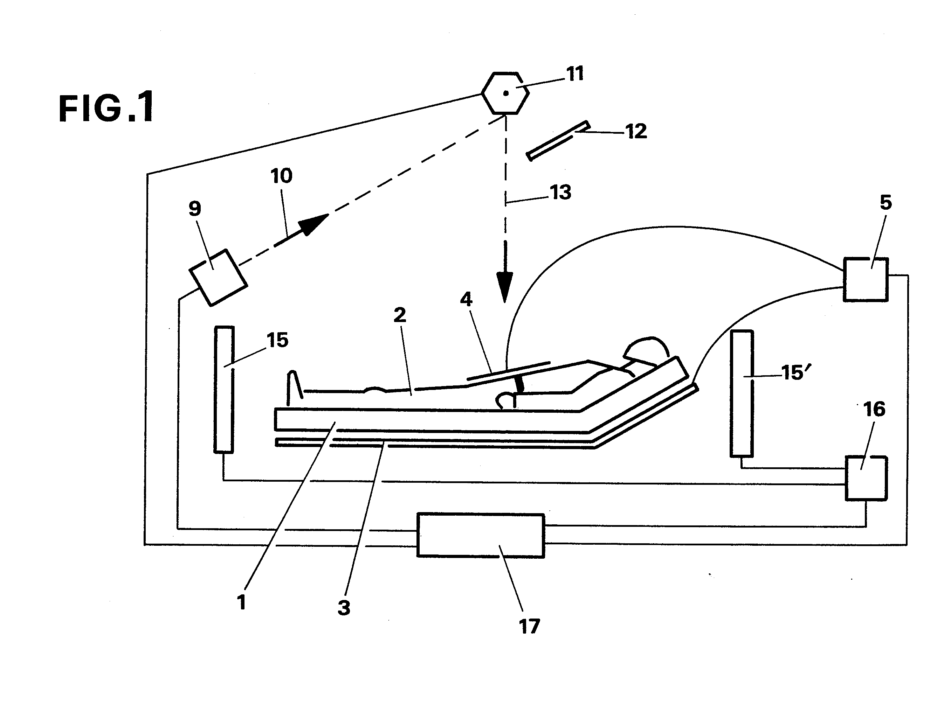

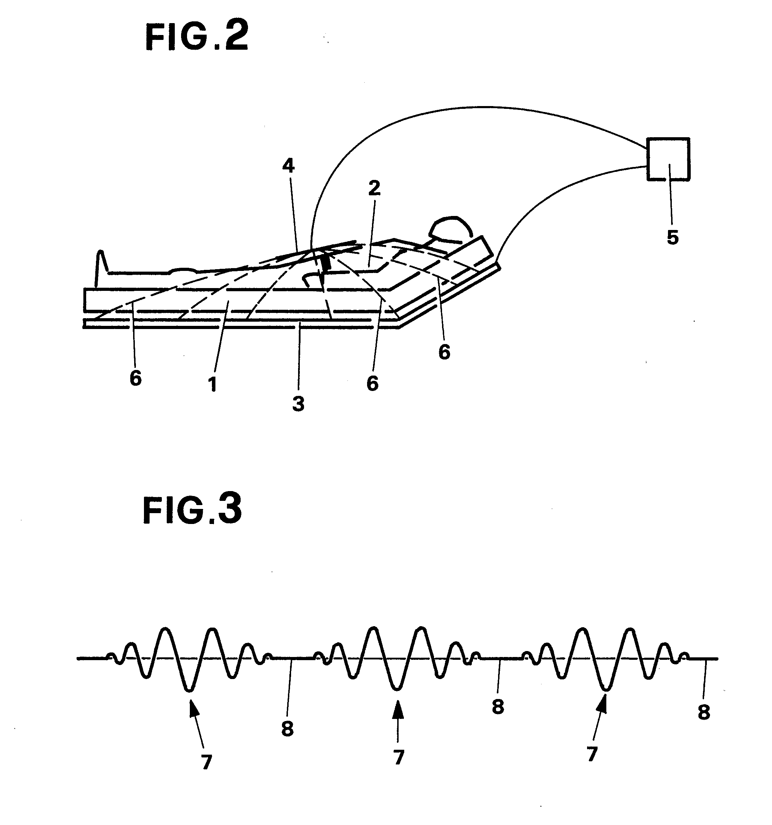

Le dispositif représenté à la figure 1 comprend un lit-condensateur 1 sur lequel on couche le patient 2 à traiter. En dessous du lit est disposée une électrode 3 dite neutre, sous toute la surface du lit. Une seconde électrode 4, active et mobile, constituée par exemple d'un maillage souple permettant d'épouser les reliefs du corps forme la deuxième armature du condensateur. Les premières et secondes électrodes 3, 4 sont reliées aux bornes d'un générateur 5 de haute fréquence. La seconde électrode 4 peut par exemple être réalisée sous la forme d'une cotte de mailles très fines (chaque maille mesurant quelques millimètres carrés), plaquée argent ou en acier inox. Comme représenté sur la figure 2, lorsque la seconde électrode 4 est placée au niveau du plexus solaire du patient à traiter, les lignes de force 6 du champ électrique se répartissent entre la surface de l'électrode active 4 et celle de l'électrode neutre 3, prenant en compte la quasi totalité du corps du patient. On peut aussi utiliser plusieurs électrodes actives dont les emplacements sont déterminés en fonction de la thérapie à appliquer.

On sait que sous l'effet des ondes haute fréquence, en utilisant en particulier la partie électrique du champ électromagnétique généré par un oscillateur haute fréquence, on obtient une mise en résonance des molécules du corps, ce qui facilite en particulier la circulation du sang par élévation de température et la régulation glandulaire du patient. Des essais ont montré que l'utilisation d'une alternance permet une bonne mise en résonance, en agissant comme un coup de maillet sur une lame de xylophone. A cet effet, le générateur 5 de haute fréquence est agencé pour produire une onde amortie croissante et décroissante, deux impulsions successives étant séparées par une zone de non activité de longueur réglable. Ce générateur permet de choisir une zone de non activité de longueur importante au début du traitement de façon à obtenir l'effet d'alternance décrit plus haut, et de diminuer ensuite la longueur de cette zone en cours de traitement, voire de la rendre de longueur nulle. Une onde amortie croissante et décroissante est représentée schématiquement à la figure 3, sur laquelle on a représenté trois impulsions 7 sous forme croissante et décroissante, séparées par des zones de non activité 8.

On choisira de préférence un générateur haute fréquence dans la gamme de fréquences de 0.2 à 300 mégahertz, d'une puissance maximum de 300 watts, la fréquence dudit générateur étant fixée par la thérapie et le tempérament du patient.

Le deuxième générateur du dispositif représenté à la figure 1 est constitué par un magnétron 9 du type à impulsions à fréquence de relaxation variable utilisant des longueurs d'ondes centimétriques, par exemple de l'ordre de 3 centimètres. Le signal 10 de ce générateur est envoyé sur un miroir tournant 11 disposé au dessus du lit-condensateur et agencé de façon à disperser ledit signal sur le corps du patient allongé sur le lit. Un écran 12 est disposé devant le miroir de façon que le signal réfléchi 13 ne puisse pas atteindre la tête du patient. Bien entendu, cet écran 12 est réglable en position. Afin de permettre un bon réglage du faisceau issu du magnétron, une lampe (non représentée) est disposée dans le même axe que le faisceau, de façon à en permettre la visualisation.

On choisira de préférence un magnétron à impulsions dans la plage de 50 hertz à 1000 hertz, pouvant atteindre une puissance de 300 watts.

Le générateur de champs magnétiques du dispositif représenté à la figure 1 peut être réalisé à l'aide d'aimants ou de bobines 15,15'. Les aimants sont placés de part et d'autre du lit-condensateur, par exemple dans le prolongement de l'axe de symétrie du patient, le côté sud aux pieds du patient allongé, le côté nord à la tête du patient. L'aimant 15' disposé à la tête du patient est rendue mobile grâce à un moteur (non représenté) dont le nombre de tours est réglable en fonction de la thérapie à appliquer. Le tout est agencé de façon à réaliser un champ magnétique faible destiné à être enregistré par les récepteurs magnétiques du corps.

Le générateur de champs magnétiques peut aussi être réalisé à l'aide de bobines, comme par exemple des bobines de Helmoltz, qui permettent d'obtenir des champs magnétiques plus homogènes. Les bobines sont disposées de la même façon que nous l'avons mentionné ci-dessus. Elles sont reliées à un générateur très basse fréquence 16, de façon à produire un champ magnétique variable en fréquence et en intensité. Le générateur 16 peut être réglé de façon à obtenir un champ magnétique continu et sensiblement uniforme, permettant d'orienter les molécules polaires dans un sens privilégié déterminé en fonction de la thérapie à appliquer.

On choisira de préférence une technologie des bobines de Helmoltz permettant d'obtenir des champs magnétiques de l'ordre de 100 Gauss.

Le générateur haute fréquence 5, le magnétron 9, le générateur 16 et le dispositif de commande du miroir tournant 11, sont reliés à un pupitre de commande 17. Une source de rayonnements ultra-violets peut compléter le dispositif, afin de prolonger les effets des champs électromagnétiques de haute fréquence dans un but de reminéralisation de l'organisme par exemple.

L'ensemble du dispositif et le patient à traiter sont installés dans une cage de Faraday (non représentée) afin d'éviter les nuisances des ondes électromagnétiques de l'environnement.

Selon une variante d'exécution du dispositif (non représentée) le patient à traiter peut aussi être placé en position assise sur une chaise-condensateur munie d'une électrode neutre placée sous la chaise. Pour le reste, le dispositif présente les mêmes caractéristiques que celles décrites plus haut.

Selon un autre mode d'exécution du dispositif, non représenté sur les dessins, le dispositif comprend un lit condensateur 1 tel que celui représenté à la figure 1, le premier générateur 5 d'ondes électromagnétiques de haute fréquence étant réalisé de la même façon que dans l'exemple ci-dessus. Par contre, le deuxième générateur 9 d'ondes électromagnétiques, et le générateur de champs magnétiques 16, 15, 15' sont disposés sur un chariot mobile, pouvant se déplacer parallèlement à la direction de l'axe longitudinal du lit. Le chariot peut par exemple être réalisé sous la forme d'un pont à roulettes se déplaçant au-dessus du lit, les aimants ou les bobines 15, 15' étant situées de chaque côté du lit de façon à constituer un champ magnétique perpendiculaire à l'axe longitudinal du lit, le deuxième générateur 9 d'ondes électromagnétiques étant placé sur le chariot de façon à pouvoir se déplacer au-dessus du lit et à produire un champ vertical, perpendiculaire au champ magnétique.

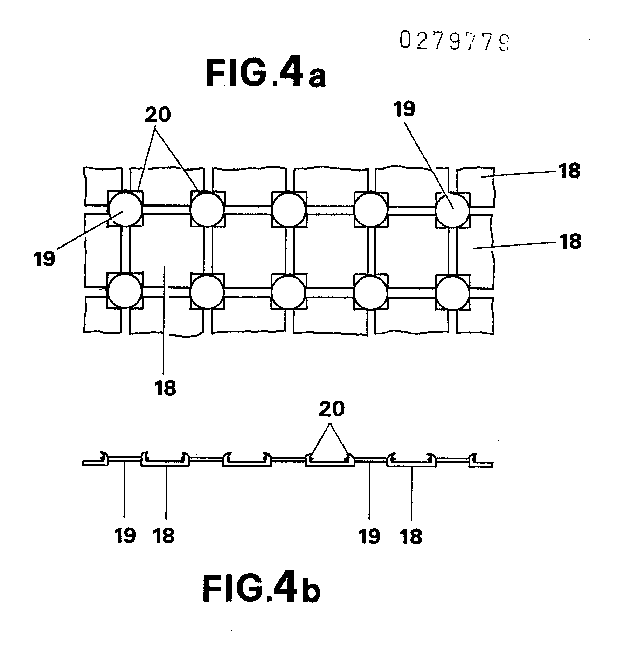

Afin d'éviter tout risque de brûlure par mauvais contact, l'électrode active du générateur haute fréquence 5 sera de préférence choisie très souple. On a représenté sur les figures 4a et 4b une telle électrode réalisée sous la forme d'une cotte de mailles à l'aide de petites plaquettes 18 en métal argenté d'environ 2 millimètres carrés de surface et d'épaisseur de 3 à 4 dixièmes de millimètres. Les bords de chaque plaquette sont recourbés pour former des éléments de crochets 20 retenant des anneaux 19 également en métal argenté. Cette cotte de mailles présente une surface très lisse et très souple qui épouse de façon quasi parfaite la surface du corps et qui permet la réalisation d'un très bon contact.

Des dispositifs annexes (non représentés) permettent la visualisation et l'enregistrement des paramètres caractéristiques d'un patient, paramètres que l'on note sur des fiches de synthèse et qui permettent le suivi correct de la thérapie.

En domaine hospitalier, on peut utiliser un champ magnétique ayant une valeur beaucoup plus élevée que ci-dessus, par exemple d'environ 600 Gauss. Les bobines génératrices peuvent être celles de Helmoltz, disposées latéralement par rapport au corps humain, ou bien ce dernier est placé dans le champ de la bobine elle-même, dans une situation identique à celle employée dans la résonance magnétique nucléaire.

Le dispositif de l'invention permet la réalisation de thérapies permettant de lutter contre un très large spectre d'affections. Il est particulièrement adapté pour le renforcement du pouvoir immunologique des patients à traiter, et plus généralement à stimuler les pouvoirs naturels de défense de l'organisme. A titre d'exemple, on donne ci-après un mode d'exécution des thérapies appliquables avec le dispositif de l'invention.

Les thérapies s'effectuent en quinze à vingt séances suivant l'état du malade. Il est demandé au départ des résultats d'analyses médicales, en particulier un hémogramme et la biochimie classique (urée, glycémie, cholestérol, etc.). Suivant le cas, une exploration des protéines plasmatiques est suggérée. Les séances durent quinze à trente minutes. Le courant de haute fréquence est au départ relativement faible. L'oscilloscope permet de se rendre compte de la forme de l'enveloppe de l'onde. On règle le générateur pour obtenir une forme bien ovoïde et de couleur uniforme. En effet, cette forme renseigne le clinicien sur l'état du patient. Une fois le courant réglé au départ, on observe une montée de ce dernier au cours de la séance. Un passage par un maximum puis une légère retombée pour arriver à une phase horizontale. Lorsque cette phase horizontale est obtenue, ce n'est plus la peine de continuer la séance. Tout a été à peu près équilibré. Les séances se déroulent soit une fois par jour, soit trois fois par semaine, puis deux fois par semaine suivant le tempérament du patient. On observe généralement à mi-parcours de la thérapie une sorte de crise ou de fatigue forte comme cela se passe dans une cure thermale. Cela prouve que les séances font effet sur l'organisme et que l'organisme réagit. En fin de thérapie, de nouvelles analyses médicales apportent les résultats objectifs obtenus, car la plupart des personnes soulignent le caractère euphorisant et l'augmentation de la lucidité intellectuelle apportée par les séances, effets qui durent assez longtemps. Deux heures avant les séances, une préparation de l'organisme est prévue par absorption d'oligo-éléments ou de phytothérapie ou d'allopathie légère. On a remarqué en effet que le pouvoir curatif de l'allopathie est multipliée par dix environ par l'application des champs électromagnétiques, ce qui permet de respecter davantage l'intégrité de l'économie humaine par diminution considérable des effets secondaires de certaines molécules (des psychotropes ou des neuroleptiques par exemple).

## Revendications

1. Dispositif pour la production de champs magnétiques et électromagnétriques biologiquement actifs, caractérisé en ce qu'il comprend un premier générateur (5) d'ondes électromagnétiques de haute fréquence sur un large spectre, un second générateur (9) d'ondes électromagnétiques utilisant les longueurs d'ondes centimétriques et un générateur (16,15,15') de champs magnétiques, le premier générateur d'ondes électromagnétiques et le générateur de champs magnétiques étant disposés et agencés pour produire des champs croisés ou parallèles en fonction de la thérapie à appliquer, le mélange des champs étant réalisé directement au niveau du corps (2) à traiter.

2. Dispositif selon la revendication 1, caractérisé en ce qu'il comporte un support (1) destiné à recevoir le corps de la personne ou de l'animal à traiter, en dessous duquel est disposée une électrode neutre (3), reliée à une borne du générateur de haute fréquence, au moins une électrode active (4) disposée sur un support mobile étant reliée à l'autre borne dudit générateur.

3. Dispositif selon la revendication 2, caractérisé en ce que le deuxième générateur est constitué par un magnétron (9) du type à impulsions à fréquence de relaxation variable, de longueurs d'ondes centimétriques, le signal (10) dudit générateur étant envoyé sur un système dispersant (11) qui le renvoie sur le corps à traiter, le générateur de champs magnétiques étant disposé de façon à produire un champ magnétique sensiblement constant dans tout le volume du corps à traiter.

4. Dispositif selon la revendication 2, caractérisé en ce que le deuxième générateur et le générateur de champs magnétiques sont solidaires d'un chariot pouvant se déplacer dans une direction parallèle à l'axe longitudinal du support (1), de façon à produire des champs perpendiculaires audit axe longitudinal du support.

5. Dispositif selon l'une des revendications 2, 3 ou 4, caractérisé en ce que le générateur de champs magnétiques comporte des aimants placés de part et d'autre du support, de façon à constituer un champ magnétique, au moins un des aimants étant mobile sous l'effet d'un moteur dont le nombre de tours est réglable en fonction de la thérapie à appliquer, ledit générateur étant agencé de façon à produire un champ magnétique faible destiné à être enregistré par les récepteurs magnétiques du corps.

6. Dispositif selon l'une des revendications 2, 3 ou 4, caractérisé en ce que le générateur de champs magnétiques comporte des bobines (15,15') placées de part et d'autre du support et reliées à un générateur basse fréquence (16) permettant la production d'un champ magnétique variable en fréquence et en intensité.

7. Dispositif selon l'une des revendications précédentes, caractérisé en ce que le champ magnétique produit est continu et sensiblement uniforme de façon à permettre l'orientation des molécules polaires dans un sens privilégié et déterminé en fonction de la thérapie à appliquer.

8. Dispositif selon l'une des revendications précédentes, caractérisé en ce que l'ensemble des éléments qui le composent sont disposés dans une cage de Faraday.

9. Dispositif selon l'une des revendications précédentes, caractérisé en ce que le générateur de haute fréquence est un oscillateur qui génère une onde haute fréquence amortie croissante et décroissante, deux impulsions successives (7) étant séparées par une zone de non activité (8) de longueur réglable.

10. Dispositif selon la revendication 9, caractérisé en ce que la zone de non activité est de longueur nulle.

**FIG.1**

**FIG.2**

**FIG.3**

# FIG.4a

# FIG.4b

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-3 100 892  (MERSMANN)<br>* Résumé; pages 2,8,114,115,118,186-189,203,204; figures 1,3-8,34,36 *<br>--- | 1-3,5-10 | A 61 N    1/42<br>A 61 N    5/02 |
| A | EP-A-0 152 963  (KRAUS)<br>* Page 5, lignes 4-31; page 7, lignes 1-4 *<br>--- | 1-3,6 | |
| A | EP-A-0 034 735  (BUSBY)<br>* Page 2, lignes 6-25; page 3, lignes 11-17; page 5, ligne 19 - page 7, ligne 17; page 10, ligne 24 - page 11, ligne 2 *<br>----- | 1-5,7 | |

DOMAINES TECHNIQUES
RECHERCHES (Int. Cl.4)

A 61 N

Le présent rapport a été établi pour toutes les revendications

| Lieu de la recherche | Date d'achèvement de la recherche | Examinateur |
|---|---|---|
| LA HAYE | 28-04-1988 | LEMERCIER D.L.L. |

EPO FORM 1503 03.82 (P0402)